(19) **Europäisches Patentamt / European Patent Office / Office européen des brevets**

(11) **EP 4 431 594 A1**

(12) **EUROPEAN PATENT APPLICATION**
published in accordance with Art. 153(4) EPC

(43) Date of publication:
**18.09.2024  Bulletin 2024/38**

(21) Application number: **22890136.9**

(22) Date of filing: **10.08.2022**

(51) International Patent Classification (IPC):
*C12N 1/12* (2006.01)      *C12P 7/40* (2006.01)
*A23K 10/16* (2016.01)     *C11B 1/02* (2006.01)
*C12R 1/89* (2006.01)

(52) Cooperative Patent Classification (CPC):
**A23K 10/16; C11B 1/02; C12N 1/12; C12P 7/40;**
C12R 2001/89

(86) International application number:
**PCT/KR2022/011959**

(87) International publication number:
**WO 2023/080400 (11.05.2023 Gazette 2023/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **08.11.2021  KR 20210152561**

(71) Applicant: **CJ CheilJedang Corporation**
**Seoul 04560 (KR)**

(72) Inventors:
 • **CHOI, Jung-Woon**
  **Seoul 04560 (KR)**
 • **KANG, Hae-Won**
  **Seoul 04560 (KR)**

 • **GWAK, Jun Seok**
  **Seoul 04560 (KR)**
 • **SHIN, Won Sub**
  **Seoul 04560 (KR)**
 • **JANG, Sunghoon**
  **Seoul 04560 (KR)**
 • **KIM, Ji Young**
  **Seoul 04560 (KR)**
 • **RYU, Ae Jin**
  **Seoul 04560 (KR)**

(74) Representative: **Reddie & Grose LLP**
**The White Chapel Building**
**10 Whitechapel High Street**
**London E1 8QS (GB)**

Remarks:
The complete document including Reference Table(s) and the Sequence Listing(s) can be downloaded from the EPO website

(54) **NOVEL STRAIN OF SCHIZOCHYTRIUM SP. WITH EASY INTRACELLULAR OIL EXTRACTION AND METHOD FOR PRODUCING OIL CONTAINING OMEGA3 USING SAME**

(57)  The present application relates to a novel strain of Schizochytrium sp. with easy intracellular oil extraction and a method for producing oil containing omega3 using same. The novel microalgae of Schizochytrium sp. according to the present invention has a high content of fat in biomass, specially a high content of unsaturated fatty acids such as docosahexaenoic acid and eicosapentaenoic acid, and thus it is very easy to extract biomass produced by itself or by cultivation and fermentation and fat components including unsaturated fatty acids from the biomass. Accordingly, the microalgae, and dried biomass and bio-oil prepared therefrom can be helpfully used as a composition for feed or a composition for food.

【FIG. 1】

**Description**

[TECHNICAL FIELD]

Cross-Reference to Related Application(s)

The present application claims the priority based on Korean Patent Application No. 10-2021-0152561 filed on November 08, 2021, and the entire contents disclosed in the documents of the corresponding Korean Patent Application are incorporated by reference as a part of the present description.

The present invention relates to a novel *Schizochytrium* sp. strain easy to extract oil in a cell and a method for producing oil containing omega3 using thereof.

[BACKGROUND ART]

Thraustochytrids survive and distribute in various environments in nature. They live in symbiosis by attaching to organisms, floats in marine environments or freshwater, or brackish environments, and are distributed in various sedimentary layers to survive. These Thraustochytrids belong to the lowest layer of the marine ecological food chain, and is also classified as organic heterotrophic protist microalgae as phytoplankton. Thraustochytrid in the natural environment is functionally responsible for circulation and purification of natural circulating elements such as sulfur, nitrogen, phosphorous, potassium, and the like. In addition, they contain polyunsaturated fatty acid (PUFA) including docosahexaenoic acid (DHA) and eicosapentaenoic acid (EPA) classified as omega-3 at a high concentration, to function as a source to the marine ecosystem.

Most higher organisms including humans cannot synthesize polyunsaturated fatty acid including docosahexaenoic acid and eicosapentaenoic acid by themselves, so they must be consumed as essential nutrients. Docosahexaenoic acid and eicosapentaenoic acid among polyunsaturated fatty acid are essential fatty acids for brain, eye tissue and nervous system, and in particular, they are known to play an important role in development of the nervous system such as eyesight and motor nerve ability in infants and prevention of cardiovascular disease, and they are the most abundant components in the structural lipid of brain.

Until now, the main source of polyunsaturated fatty acid is fish oil extracted from oil of blue fish such as mackerel, saury, tuna, horse mackerel, sardine, herring and the like, and this is very useful as a fish culture feed such as initial feed for seawater fish. Although extraction and intake of polyunsaturated fatty acid from fish oil has been industrially developed, there are also disadvantages. The quality of fish oil varies depending on the species of fish, season and fishing location, and it is generated through fishing, so it is difficult to continuously supply it. In addition, there are limitations in the producing process and production due to the problem of contamination by heavy metals and organic chemicals comprised in fish oil, problem of oxidation of double bonds during the processing process as well as peculiar fishy smell of fish oil, and the like.

In order to solve these problems, recently, research on a method for production of polyunsaturated fatty acid including docosahexaenoic acid and eicosapentaenoic acid by microbial culture is being conducted. In particular, microalgae may provide several advantages over fish oil in addition to the ability to newly synthesize fatty acid naturally. It can be stably supplied through culturing on an industrial scale, and it enables production of biomass having a relatively constant biochemical composition. Unlike fish oil, lipid produced by microalgae do not have any unpleasant odor. Furthermore, it has a simpler fatty acid composition compared to fish oil, which facilitates the steps to isolate major fatty acid.

Based on these advantages, recently, research and industrialization on production of polyunsaturated fatty acid comprising omega-3 unsaturated fatty acid ($\omega$)unsaturated fatty acid) such as docosahexaenoic acid (DHA), eicosapentaenoic acid (EPA), arachidonic acid (ARA), docosapentaenoic acid (DPA), and $\alpha$-linolenic acid, and the like, are progressing very rapidly, and they are mainly production of polyunsaturated fatty acid by *Thraustochytrium* sp. and *Schizochytrium* sp. microorganisms which are a kind of marine microalgae. For example, a method for producing omega-3 polyunsaturated fatty acid using *Schizochytrium* sp. ATCC20888 and *Schizochytrium* sp. PTA10208 which are *Schizochytrium* sp. microorganisms (U.S. Patent No. 5,130,242), and additionally, a method for producing docosahexaenoic acid and eicosapentaenoic acid using *Thraustochytrium* sp. ATCC10212 (Thraustochytrium sp. PTA10212) which is a Thraustochytrid series *Thraustochytrium* sp. microorganism is disclosed.

[DISCLOSURE]

[TECHNICAL PROBLEM]

One embodiment of the present invention provides a novel *Schizochytrium* sp. microalgae. In one specific embodiment, the novel *Schizochytrium* sp. microalgae may be a *Schizochytrium* sp. CD01-1821 microalgae (Accession number KCTC14660BP).

Another embodiment of the present invention provides biomass or bio-oil derived from the *Schizochytrium* sp. microalgae.

Other embodiment of the present invention provides a feed composition comprising the *Schizochytrium* sp. microalgae-derived biomass, bio-oil or a combination thereof.

Other embodiment of the present invention provides a food composition comprising the *Schizochytrium* sp. microalgae-derived biomass, bio-oil or a combination thereof.

Other embodiment of the present invention provides a method for producing biomass or bio-oil derived from the *Schizochytrium* sp. microalgae.

Other embodiment of the present invention provides a use for producing biomass or bio-oil of the *Schizochytrium* sp. microalgae.

Other embodiment of the present invention provides a use for producing a feed composition or food composition of the *Schizochytrium* sp. microalgae.

[TECHNICAL SOLUTION]

Each description and embodiment disclosed in the present application can be applied to each of other description and embodiments. In other words, all combinations of various elements disclosed in the present application fall within the scope of the present invention. In addition, it cannot be seen that the scope of the present invention is limited by the detailed description described below. Furthermore, those skilled in the art will recognize or confirm many equivalents to specific aspects of the present invention described in the present application using only a common experiment. In addition, such equivalents are intended to be included in the present invention.

One embodiment of the present invention provides a novel *Schizochytrium* sp. microalgae.

The term used in the present description, "Thraustochytrid" means an order *Thraustochytriales* microalgae. In addition, the term used in the present description, *"Schizochytrium* sp." is one of the genus names belonging to the family *Thraustochytriaceae* of the order *Thraustochytriales,* and may be interchangeably used with the term *"Schizochytrium* sp. (genus *Schizochytrium*)"*. Furthermore, the term "microalgae" means an organism that cannot be seen with a naked eye and can only be seen through a microscope among photosynthetic plants with chlorophyll, and lives freely floating in water, and is also called phytoplankton. There are various types of the microalgae, and since photosynthesis is impossible, even strains that grow only through heterotrophs are included.

As one embodiment in the present invention, after collecting environmental samples from the coast areas to obtain pure isolated colonies of the Thraustochytrid series, as a result of analysis of total lipid and fatty acids, CD01-1821 and CD01-1822 with excellent intracellular DHA content two kinds of strains were obtained. As a result of culture evaluation, it was confirmed that the CD01-1821 strain was more useful for a scale-up process, as it had higher total production of biomass and total oil content under the same fermentation condition.

Therefore, in the present invention, the novel *Schizochytrium* sp. microalgae may be *Schizochytrium* sp. CD01-1821 microalgae (Accession number KCTC14660BP).

In addition, the *Schizochytrium* sp. strain may have a 18s rRNA nucleotide sequence of SEQ ID NO: 1, but not limited thereto. For example, the *Schizochytrium* sp. microalgae may have 18S rRNA consisting of a nucleotide sequence showing the sequence identity of 80% or more, 85% or more, 90% or more, 95% or more, 98% or more, or 99% or more to the nucleotide sequence of SEQ ID NO: 1, but not limited thereto.

The term used in the present description, "docosahexaenoic acid (DHA)" is one of polyunsaturated fatty acids having the chemical formula of $C_{22}H_{32}O_2$, and corresponds to omega-3 fatty acid together with alpha-linolenic acid ($\alpha$-linolenic acid: ALA) and eicosapentaenoic acid (EPA), and the common name is cervonic acid, and it may also be expressed as 22:6 n-3 as an abbreviation.

The term used in the present description, "eicosapentaenoic acid (EPA)" is one of polyunsaturated fatty acids having the chemical formula of $C_{20}H_{30}O_2$, and corresponds to omega-3 fatty acid together with ALA and DHA, and it may also be expressed as 20:5 n-3 as an abbreviation.

The *Schizochytrium* sp. microalgae may produce and/or comprise DHA of 35 to 60 % by weight based on the total weight of fatty acid. For example, the *Schizochytrium* sp. microalgae may produce DHA of 40 to 65 % by weight, 45 to 65 % by weight, 50 to 65 % by weight, 40 to 60 % by weight, 45 to 60 % by weight, 50 to 60 % by weight, or 55 to 60 % by weight, based on the total weight of fatty acid.

The *Schizochytrium* sp. microalgae may produce and/or comprise EPA of 0.1 to 2 % by weight based on the total weight of fatty acid. For example, the *Schizochytrium* sp. microalgae may produce EPA of 0.2 to 2 % by weight, 0.2 to 1.5 % by weight, 0.2 to 1 % by weight, 0.3 to 2 % by weight, 0.3 to 1.5 % by weight, 0.3 to 1 % by weight, 0.5 to 2 % by weight, 0.5 to 1.5 % by weight, 0.5 to 1 % by weight, 0.5 to 0.9 % by weight or 0.6 to 0.8 % by weight, based on the total weight of fatty acid.

Another aspect of the present invention provides biomass or bio-oil derived from the *Schizochytrium* sp. microalgae, comprising the *Schizochytrium* sp. CD01-1821 microalgae, a culture of the microalgae, a dried product of the culture, or a lysate of the dried product.

The description of the *Schizochytrium* sp. microalgae is as described above.

The term used in the present description, "biomass" means an energy source of bioenergy, that is, a living organism such as a plant, an animal, a microorganism, and the like, and also means the weight or energy amount of a specific living organism present in a unit time and space ecologically. In addition, the biomass comprises a compound secreted by a cell, but not limited thereto, and may contain a cell and/or a content in the cell as well as an extracellular material. In the present application, the biomass may be the *Schizochytrium* sp. microalgae itself, a culture thereof, a dried product thereof, a lysate thereof, or a product produced by culturing or fermenting the microalgae, or may be a concentrate or dried product of the biomass, but not limited thereto.

The "culture" of the *Schizochytrium* sp. microalgae refers to a product produced by culturing the microalgae, and specifically, it may be a culture solution comprising the microalgae or a culture filtrate from which the microalgae is removed from the culture solution, but not limited thereto. The "dried product" of the *Schizochytrium* sp. microalgal culture is that moisture is removed from the microalgal culture, and for example, it may be in a dried microbial cell of the microalgae, but not limited thereto. Moreover, the "lysate" of the dried product collectively calls the result of lysing the dried product from which moisture is removed from the microalgal culture, and for example, it may be a dried microbial cell powder, but not limited thereto. The culture of the *Schizochytrium* sp. microalgae may be prepared according to a culturing method known in the art by inoculating the microalgae to a microalgal culture medium, and the dried product of the culture and lysate thereof may be also prepared according to a method for processing or drying of a microalgae or culture solution known in the art.

The biomass derived from the *Schizochytrium* sp. microalgae may comprise crude fat of 40 to 85 % by weight, 45 to 80 % by weight, 50 to 75 % by weight, 50 to 70 % by weight, 54 to 66 % by weight based on the total weight of biomass.

The biomass derived from the *Schizochytrium* sp. microalgae may comprise crude protein of 5 to 25 % by weight, 5 to 20 % by weight, 10 to 20 % by weight, or 15 to 20 % by weight based on the total weight of biomass.

The biomass derived from the *Schizochytrium* sp. microalgae may comprise DHA of 35 % by weight or more, or 35 to 60 % by weight based on the total weight of fatty acid, and may comprise EPA of 0.1 % by weight or more, or 0.1 to 2 % by weight based on the total weight of fatty acid, and may comprise palmitic acid of 30 to 40 % by weight or more based on the total weight of fatty acid.

The *Schizochytrium* sp. microalgae has a high oil content a high oil content and, in particular, a high omega-3 content, so that the culture time in the complex sugar can be shortened. Therefore, it is advantageous for the scale-up process.

The "oil content" may be interchangeably used with "crude fat content".

The biomass may be prepared by the method of preparation of biomass derived from a *Schizochytrium* sp. microalgae according to one aspect.

Other aspect of the present invention provides a composition comprising the *Schizochytrium* sp. CD01-1821 microalgae, a culture of the microalgae, a dried product of the culture, or a lysate of the dried product.

The composition may comprise the *Schizochytrium* sp. microalgae-derived biomass, bio-oil, or a combination thereof.

Other aspect of the present invention provides a feed composition comprising biomass derived from the *Schizochytrium* sp. CD01-1821 microalgae, or a concentrate or dried product of the biomass.

The description of the *Schizochytrium* sp. microalgae, biomass, culture of the microalgae, dried product of the culture, and lysate of the dried product are as described above.

The concentrate or dried product of biomass may be prepared according to a method for treatment, concentration or drying of microbial biomass known in the art.

The term used in the present description, "bio-oil" means oil obtained from biomass by biological, thermochemical and physicochemical extraction processes, and in the present application, the prepared bio-oil may contain polyunsaturated fatty acid, and specifically, may contain DHA and EPA, but not limited thereto.

In the present description, the bio-oil may comprise an extract of biomass.

As the method for preparing a bio-oil extract, a method for utilizing enzyme such as protease, cellulase, pectinase or chitinase, or the like according to a method for lysing or dissolving a cellular membrane or cell wall component, a method for physically lysing a cellular membrane or cell wall component using a homogenizer, a ultrasonicator, bead processing, and the like, a method for extracting by intracellular permeation by directly adding a solvent, a solvent-free extraction process of separating through a centrifugation process after various lysing processes, and the like may be used, but not limited thereto.

The composition may be in a form of solution, powder or suspension, but not limited thereto. The composition may be for example, a food composition, feed composition or feed additive composition.

The term used in the present description, "feed composition" refers to food fed to an animal. The feed composition refers to a substance supplying an organic or inorganic nutrient necessary for maintaining life of an animal or producing meat, milk, and the like. The feed composition may further comprise a nutritional component necessary for maintaining life of an animal or producing meat, milk, and the like. The feed composition may be produced as various types of feed known in the art, and specifically, may comprise concentrated feed, roughage and/or special feed.

The term used in the present description, "feed additive" includes substances added to feed on a purpose of various effects such as nutrient supplementation and weigh loss prevention, enhancement of digestibility of fiber in feed, improvement of oil quality, reproductive disorder prevention and fertility rate improvement, prevention of high temperature stress in summer, and the like. The feed additive of the present application correspond to supplementary feed under the Feed Management Act, and may further comprise mineral matter preparations such as sodium hydrocarbon, bentonite, magnesium oxide, complex mineral, and the like, mineral preparations which are trace mineral matters such as zinc, copper, cobalt, selenium and the like, vitamin preparations such as keratin, vitamin E, vitamins A, D and E, nicotinic acid, vitamin B complex, and the like, protective amino acid preparations such as methionine, lysine, and the like, protective fatty acid preparations such as fatty acid calcium salt, and the like, live cells and yeast agents such as probiotics (bacteria preparation), yeast culture, mold fermented product, and the like.

The term used in the present description, "food composition" includes all forms of functional food, nutritional supplement, health food and food additives, and the like, and the above types of food compositions may be prepared in various forms according to a common method known in the art.

The composition of the present application may further comprise grains such as milled or crushed wheat, oats, barley, corn and rice; plant protein feed, for example, feed having soybean and sunflower as a main component; animal protein feed, for example, blood meal, meat meal, bone meal and fish meal; sugar and dairy products, for example, dried components consisting of various kinds of powdered milk and whey powder, and in addition thereto, may further comprise a nutritional supplement, a digestion and absorption enhancer, a growth promoter, and the like.

The composition of the present application may be administered alone or administered in combination with other feed additive among an edible carrier. In addition, the composition may be directly mixed as top dressing or to feed or be easily administered as an oral formulation separate to feed into an animal. When the composition is administered separately to feed, it may be prepared as an intermediate release or sustained-release formulation, in combination of a pharmaceutically acceptable edible carrier as well known in the art. This edible carrier may be a solid or liquid, for example, corn starch, lactose, sucrose, soybean flake, peanut oil, olive oil, sesame oil and propylene glycol. When a solid carrier is used, the composition may be top dressing in a tablet, capsule, powder, troche or lozenge or undispersible form. When a liquid carrier is used, the composition may be a formulation of gelatin soft capsule, or syrup or suspension, emulsion or solution.

The composition of the present application may contain, for example, a preservative, stabilizer, wetting agent or emulsifier, cryoprotectant, or excipient, or the like. The cryoprotectant may be one or more selected from the group consisting of glycerol, trehalose, maltodextrin, nonfat dry milk and starch.

The preservative, stabilizer or excipient may be comprised in the composition in an effective dose sufficient for reducing deterioration of the *Schizochytrium* sp. microalgae comprised in the composition. In addition, the cryoprotectant may be comprised in the composition in an effective dose sufficient for reducing deterioration of the *Schizochytrium* sp. microalgae comprised in the composition when the composition is in a dried state.

The composition may be used by be adding to feed of an animal by sedimentation, spraying or mixing.

The composition of the present invention may be applied to various animal diets including mammals, birds, fish, crustaceans, cephalopods, reptiles and amphibians, but not limited thereto. For example, the mammal may include a pig, cow, sheep, goat, experimental rodent or pet, and the bird may include a poultry, and the poultry may include a chicken, turkey, duck, goose, pheasant or quail, or the like, but not limited thereto. In addition, the fish may include commercial livestock fish and fry thereof, ornamental fish, and the like, and the crustacean may include shrimp, barnacles, and the like, but not limited thereto. Furthermore, the composition may be applied to the diet of zooplankton, a rotifer.

Other aspect of the present invention provides a method for producing biomass derived from a *Schizochytrium* sp. microalgae, comprising culturing the *Schizochytrium* sp. CD01-1821 microalgae; and recovering biomass containing docosahexaenoic acid from the microalgae, a dried product thereof, or a lysate thereof.

The description of the *Schizochytrium* sp. microalgae, biomass, culture of the microalgae, dried product of the culture, and lysate of the dried product are as described above.

The term used in the present description, "culture" means to grow the microalgae in an appropriately controlled environmental condition. The culturing process of the present application may be conducted according to a suitable medium and a culture condition known in the art. This culturing process may be easily adjusted and used by those skilled in the art according to the selected microalgae.

Specifically, the culturing of the *Schizochytrium* sp. microalgae of the present application may be performed under a heterotrophic condition, but not limited thereto.

The term used in the present description, "heterotrophic" is a nutritional method depending on an organic matter obtained from an energy source or nutrient source outside the body, and is a term corresponding to autotrophic, and it may be interchangeably used with a term 'dark culture'.

The culturing a *Schizochytrium* sp. microalgae is not particularly limited thereto, but may be performed by known batch culturing method, continuous culture method, fed-batch culturing method, and the like. Any medium and other culturing condition used in the culturing of the microalgae of the present application may be used without particular limitation as long as it is a medium used for culturing common microalgae. Specifically, the microalgae of the present application may be cultured by adjusting temperature, pH and the like, under an aerobic condition in a common medium containing a suitable carbon source, a nitrogen source, a phosphorus source, an inorganic compound, an amino acid and/or a vitamin, and the like.

Specifically, an appropriate pH (for example, pH 5 to 9, specifically, pH 6 to 8, most specifically, pH 6.8) may be adjusted using a basic compound (e.g.: sodium hydroxide, potassium hydroxide or ammonia) or acidic compound (e.g.: phosphate or sulfate), but not limited thereto.

In addition, in order to maintain the aerobic condition of the culture, oxygen or oxygen-containing gas may be injected into the culture, or in order to maintain anaerobic and microaerobic conditions, nitrogen, hydrogen or carbon dioxide may be injected without injection of gas, but not limited thereto.

Furthermore, the culturing temperature may be maintained at 20 to 45°C or 25 to 40°C, and may be cultured for about 10 to 160 hours, but not limited thereto. In addition, during the culturing, an antifoaming agent such as fatty acid polyglycol ester may be used to inhibit bubble formation, but not limited thereto.

The carbon source comprised in the medium used in the culturing the *Schizochytrium* sp. microalgae may be any one or more selected from the group consisting of glucose, fructose, maltose, galactose, mannose, sucrose, arabinose, xylose and glycerol, but any carbon source used for culturing microalgae is not limited thereto.

The nitrogen source comprised in the medium used in the culturing the *Schizochytrium* sp. microalgae may be (i) any one or more of organic nitrogen sources selected from the group consisting of yeast extract, beef extract, peptone and tryptone, or ii) any one or more of inorganic nitrogen sources selected from the group consisting of ammonium acetate, ammonium nitrate, ammonium chloride, ammonium sulfate, sodium nitrate, urea and MSG (Monosodium glutamate), but any nitrogen source used for culturing a microalgae is not limited thereto.

The medium used in the culturing the *Schizochytrium* sp. microalgae, may separately comprise or mix and comprise potassium dihydrogen phosphate, dipotassium hydrogen phosphate, as a phosphorus source, and a sodium-containing salt corresponding thereto, and the like, but not limited thereto.

The recovering biomass from the microalgae, culture of the microalgae, dried product of the culture, or lysate of the dried product may collect targeted biomass using an appropriate method known in the art. For example, centrifugation, filtration, anion exchange chromatography, crystallization and HPLC and the like may be used, and it may further comprise a purification process.

Other aspect of the present invention provides a method for producing bio-oil derived from a *Schizochytrium* sp. microalgae, comprising culturing the *Schizochytrium* sp. CD01-1821 microalgae; and recovering biomass containing docosahexaenoic acid from the microalgae, a dried product thereof, or a lysate thereof.

The description of the *Schizochytrium* sp. microalgae, bio-oil, culture of the microalgae, dried product of the culture, and lysate of the dried product, and culturing the microalgae are as described above.

The recovering lipid from the microalgae, culture of the microalgae, dried product of the culture, or lysate of the dried product may collect targeted lipid using an appropriate method known in the art. For example, centrifugation, filtration, anion exchange chromatography, crystallization and HPLC and the like may be used, and it may further comprise a purification process.

For example, lipid and lipid derivatives such as fat aldehyde, fat alcohol and hydrocarbon (for example, alkane) may be extracted with a hydrophobic solvent such as hexane. The lipid and lipid derivatives may be also extracted using a method for liquefaction, oil liquefaction and supercritical $CO_2$ extraction, and the like. In addition, a known microalgal lipid recovery method includes for example, a method i) for harvesting cells by centrifugation and washing with distilled water, and then drying by freeze-drying, and ii) for pulverizing the obtained cell powder, and then extracting lipids with n-hexane (Miao, X and Wu, Q, Biosource Technology (2006) 97:841-846).

Other aspect of the present invention provides a use for producing biomass or bio-oil of the *Schizochytrium* sp. CD01-1821 microalgae, a culture of the microalgae, a dried product of the culture, or a lysate of the dried product.

The *Schizochytrium* sp. microalgae, biomass, culture of the microalgae, dried product of the culture, and lysate of the dried product are as described above.

Other embodiment of the present invention provides a use for producing a feed composition or food composition of the *Schizochytrium* sp. CD01-1821 microalgae, a culture of the microalgae, a dried product of the culture, or a lysate of the dried product.

The *Schizochytrium* sp. microalgae, biomass, culture of the microalgae, dried product of the culture, and lysate of the dried product are as described above.

[ADVANTAGEOUS EFFECTS]

The novel Thraustochytrid series microalgae of the present invention have a high fat content in biomass and have a high content of unsaturated fatty acids such as docosahexaenoic acid and eicosapentaenoic acid among them, and it is very easy to extract biomass produced by itself or by culture and fermentation and fat components including unsaturated fatty acids from biomass. Accordingly, the microalgae, the biomass dried product and bio-oil produced therefrom can be usefully used as a composition for feed or a composition for food, or the like.

[BRIEF DESCRIPTION OF THE DRAWINGS]

FIG. 1 is a schematic diagram showing a process of separating a *Thraustochytrid* series microalgal strain.
FIG. 2 is a drawing which shows the result of analyzing the total lipid content, and the content of docosahexaenoic acid (DHA) and eicosapentaenoic acid (EPA) among omega-3 of 29 kinds of separated *Thraustochytrid* series microalgae.
FIG. 3 is a photograph observing the wild-type *Schizochytrium* sp. strain CD01-1821 with an optical microscope.

[MODE FOR INVENTION]

Hereinafter, the present invention will be described in more detail by examples. However, these examples are intended to illustratively described one or more specific embodiments, and the scope of the present invention is not limited by these examples.

**Example 1. Isolation of Thraustochytrid series microalgae**

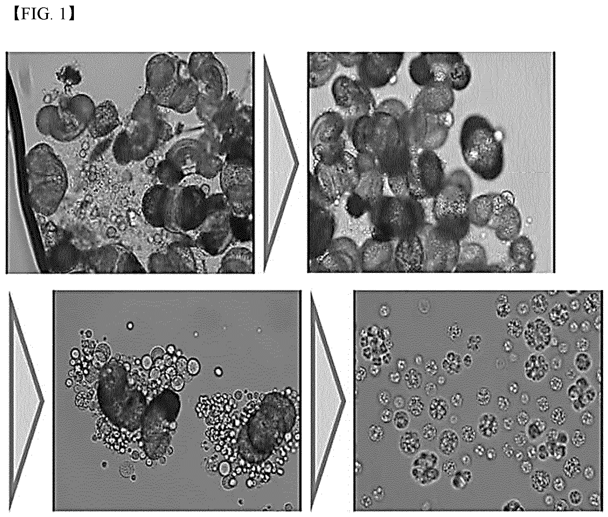

In order to isolate a Thraustochytrid series microalgae, environmental samples in the form of seawater, leaves and sediments were collected from a total of 40 areas along Korean west coast areas, Seocheon, Gunsan, Buan and Yeonggwang-gun areas. Sampling was carried out centered on a specific area where organic sediments were developed and observed, and the collected environmental samples were transported to the laboratory environment within 7 days, and an operation for removing other pollution sources such as bacterial microorganisms and fungi and protists except for Thraustochytrid series microalgae was progressed. Through continuous microscopic examination, Thraustochytrid series microalgal cells were isolated focusing on samples which showed characteristic morphology and formed an observable zoospore within the life cycle, or constituted the ectoplasmic network generated during the developmental stage (FIG. 1). As a separation and culturing medium usable in the isolated process, a modified YEP medium (Yeast extract 0.1 g/L, peptone 0.5 g/L, $MgSO_4.7H_2O$ 2 g/L, Sea salt 50 g/L, $H_3BO_3$ 5.0 mg/L, $MnCl_2$ 3.0 mg/L, $CuSO_4$ 0.2 mg/L, $NaMo_4.2H_2O$ 0.05 mg/L, $CoSO_4$ 0.05 mg/L, $ZnSO_4.7H_2O$ 0.7 mg/L, Agar 15 g/L) was utilized. Pure isolated colonies from which the pollution sources were removed through several times of isolation and subculturing processes could be obtained, and the isolated colonies were under a pollution source control and removal process again in a solid medium including an antibiotic cocktail mix solution (Streptomycin sulfate 0-50 mg/L, Ampicillin 0-30 mg/L, Penicillin G 0-30 mg/L, Kanamycin sulfate 0-30 mg/L), and thereby, pure separable colonies were obtained.

**Example 2. Culturing evaluation of isolated microalgae and superior strain selection**

Culturing evaluation for the pure isolated colonies in Example 1 was performed, and through this, a superior strain was selected.
Specifically, the pure isolated colonies in Example 1 were cultured using a modified GGYEP medium (glucose 5 g/L, glycerol 5 g/L, yeast extract 0.1 g/L, peptone 0.5 g/L, $MgSO_4.7H_2O$ 2 g/L, sea salt 50 g/L, $H_3BO_3$ 5.0 mg/L, $MnCl_2$ 3.0 mg/L, $CuSO_4$ 0.2 mg/L, $NaMo_4.2H_2O$ 0.05 mg/L, $CoSO_4$ 0.05 mg/L, $ZnSO_4.7H_2O$ 0.7 mg/L) in a 250 mL flask under the condition of 10-35°C, 100-200 rpm for about 2 days. Based on the progressed culturing result, 29 kinds of microalgae that could grow at a temperature of 30°C or higher, and had an excellent growth rate and could secure a microbial cell amount were selected. For the selected microalgal strains, 500 ml flask scale culturing of the modified GYEP medium including glucose of 30 g/L as a carbon source and culturing condition of 30°C, 150rpm was performed for 2 days. After confirming that all of the input carbon sources were consumed in the culturing environment for 2 days, the entire culturing solution was recovered and dried overnight in a dry oven at 60°C to obtain biomass.
In order to analyze the lipid and polyunsaturated fatty acid contents of the cultured microalgal microbial cells, the method as below was used, and the microalgae-derived fatty acid-containing oil utilizing the dried microbial cells was measured by the following method. A process of adding 8.3 M hydrochloric acid solution (HCl) to 5 g of the dried microbial cells, and then hydrolyzing the cell walls of the microalgal microbial cells at 80°C, and then adding ethyl ether 30 mL and petroleum ether 20 mL and mixing for 30 seconds, and then centrifuging was repeated 3 times or more. The separated solvent layer was recovered, and transferred to a pre-weighed round flask, and then the solvent was removed through nitrogen purging, and the constant weight was cooled in a desicator. By measuring the weight of the dried oil by subtracting the weight of the empty flask from the weight of the flask after drying, the total oil content was calculated. The content of docosahexaenoic acid (DHA) comprised in the oil was shown by pre-treating with methanolic 0.5N NaOH and 14% trifluoroborane methanol ($BF_3$) and measuring by gas chromatography.

[Calculation formula 1]

Total oil content (%) = (*oil g/dried microbial cell amount g) X 100

*oil g: flask weight after acid hydrolysis and solvent removal - ball flask weight

The "biomass" of Table 1 below means a concentration of the microbial cell in the culture solution, and it may be interchangeably used with DCW (dry cell weight) of Table 2 below.

[Table 1]

|  | Total oil (%/biomass) | Fatty acid content (%/TFA) | |
|---|---|---|---|
|  |  | DHA | EPA |
| 1811 | 40.01 | 19.46 | 1.72 |
| 1812 | 36.03 | 17.90 | 1.80 |
| 1813 | 34.21 | 19.40 | 2.10 |
| 1814 | 38.67 | 18.90 | 1.98 |
| 1815 | 28.89 | 17.90 | 2.92 |
| 1816 | 23.84 | 17.64 | 3.38 |
| **1821** | **31.53** | **57.25** | **0.71** |
| **1822** | **43.18** | **58.68** | **0.53** |
| 1831 | 29.80 | 29.38 | 1.98 |
| 1832 | 31.50 | 33.35 | 1.61 |
| 1833 | 22.36 | 37.26 | 2.83 |
| 1834 | 23.44 | 36.12 | 2.22 |
| 1835 | 39.03 | 29.85 | 0.70 |
| 1836 | 21.73 | 35.77 | 2.76 |
| 1837 | 18.39 | 33.30 | 2.96 |
| 1838 | 19.81 | 38.77 | 2.76 |
| 1839 | 20.76 | 37.91 | 3.06 |
| 18310 | 20.65 | 39.93 | 2.85 |
| 18311 | 22.96 | 40.17 | 2.70 |
| 18312 | 18.26 | 39.27 | 3.31 |
| 18313 | 21.99 | 36.68 | 2.69 |
| 1841 | 26.33 | 19.40 | 1.06 |
| 1842 | 35.53 | 21.75 | 1.38 |
| 1843 | 34.83 | 18.67 | 1.02 |
| 1844 | 29.74 | 23.92 | 1.65 |

(continued)

| | Total oil (%/biomass) | Fatty acid content (%/TFA) | |
|---|---|---|---|
| | | DHA | EPA |
| 1845 | 33.23 | 19.89 | 0.89 |
| 1846 | 28.23 | 20.63 | 1.13 |
| 1847 | 31.37 | 18.56 | 0.91 |
| 1848 | 30.51 | 42.74 | 1.06 |
| 1849 | 29.19 | 20.46 | 1.50 |
| 18410 | 24.63 | 25.17 | 1.84 |
| 18411 | 21.93 | 25.75 | 1.96 |

(In the table, TFA means total fatty acid, and may be interchangeably used with "crude fat content" or "crude fat amount" or "total lipid".)

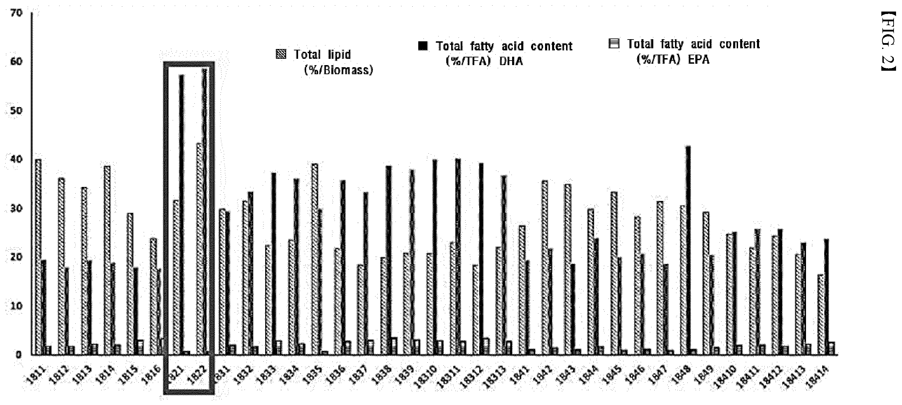

As a result, as shown in Table 1 and FIG. 2, the intracellular DNA content was shown very high in the 2 kinds of strains of CD01-1821 and CD01-1822.

As the result of the fatty acid analysis, culturing evaluation was performed in a 5L scale culture medium for the 2 kinds of strains of CD01-1821, CD01-1822 with the excellent intracellular DNA content. For seed culture, they were cultured using a sterilized MJW02 medium (glucose 30 g/L, $MgSO_4.7H_2O$ 3.0 g/L, $Na_2SO_4$ 15 g/L, NaCl 0.8 g/L, yeast extract 1.0 g/L, $MSG.1H_2O$ 1.0 g/L, $NaNO_3$ 1.0 g/L, $KH_2PO_4$ 0.8 g/L, $K_2HPO_4$ 1.5 g/L, $CaCl_2$ 0.5 g/L, vitamin mixed solution 10 ml/L) in a 500 mL flask under the condition of 30°C, 150 rpm for about 24 hours. The seed cultured flask was aliquoted and inoculated in a 5L culture medium. A glucose carbon source of 28% compared to the total culturing solution was supplied and culturing was progressed for about 72 hours, and culturing was performed in a sterilized MJW02 medium under the culturing environment condition of 30°C, 500 rpm, 1.5 vvm, pH 5-8.

[Table 2]

| | *Schizochytrium* sp. CD01-1821 | *Schizochytrium* sp. CD01-1822 |
|---|---|---|
| Culturing time (hr) | 71.5 | 71.5 |
| O.D (680nm) | 154.2 | 103 |
| DCW (g/L) | 139.5 | 103 |
| Crude fat content (%) | 58.6 | 49.7 |

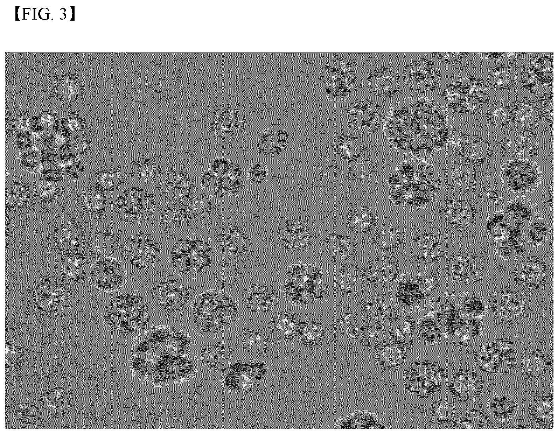

As a result, as shown in Table 2, it was confirmed that the CD01-1821 strain was more useful for a scale-up process, as it had higher total production of biomass and crude fat amount under the same fermentation condition than the CD01-1822 strain. Accordingly, the CD01-1821 strain was selected and used for strain sequence identification and additional strain development. The type of the selected CD01-1821 strain was observed using an optical microscope and shown in FIG. 3.

**Example 3. Confirmation of culturing characteristics of CD01-1821 strain under complex carbon source condition**

In heterotrophic microorganism-based fermentation, a glucose component is mainly used as a raw material for a carbon source. At this time, glucose is a monosaccharide in a refined form of 90% or more, and the cost is generated higher during industrial scale fermentation compared to other carbon source raw material components. In order to utilize an inexpensive carbon source raw material and obtain price competitiveness through this, it is important to discover a strain that can be used for fermentation by a microorganism and can be cultured normally from the inexpensive carbon source component, other than purified glucose.

Therefore, fermentation culturing evaluation was performed in raw sugar having glucose, fructose or sucrose as a main component for the CD01-1821 strain selected in Example 2 and CJM01 (KR 10-2100650 B1) strain to confirm culturing characteristics. The culturing was performed in a 30L culture medium, and based on the modified MJW02 medium, experiments were conducted with raw sugar lysates comprising glucose 450 g/L, a mixture of glucose 225 g/L and fructose 225 g/L, glucose 225 g/L, fructose 220 g/L and sulfate 1.51 g/L, respectively. The culturing condition was set same as the condition of 30°C, 500 rpm, 1.5 vvm, pH 5-8, and a carbon source of 35% of the total culturing solution was supplied, respectively.

[Table 3]

| Carbon source | Schizochytrium sp. CD01-1821 | | | Thraustochytrium sp. CJM01 | | |
|---|---|---|---|---|---|---|
| | Glucose | Glucose+fructose mixture | Raw sugar (Sucrose) lysate | Glucose | Glucose+fructose mixture | Raw sugar (Sucrose) lysate |
| Culturing time (hr) | 54.7 | 56.1 | 7.3 | 60 | 66.83 | 83.3 |
| O.D (680nm) | 169.3 | 143.7 | 177.9 | 152.7 | 180.4 | 155.7 |
| DCW (g/L) | 163 | 160 | 161 | 130 | 150 | 138 |
| Crude fat amount (%) | 60.7 | 60.1 | 60.3 | 61.2 | 61.7 | 59.3 |

As a result, as shown in Table 3, the CD01-1821 strain showed the total biomass production and crude fat amount at an equivalent or higher level in fermentation under a fructose mixture or raw sugar lysate medium condition, not a glucose single component. On the other hand, the CJM01 strain showed a diauxic growth form of dualized carbon source consumption and cell growth pattern as a sugar component other than the glucose component is added in the medium and showed a phenomenon that the total culturing time was prolonged. Through the corresponding experiment result, it could be confirmed that the CD01-1821 strain had scale-up fermentation possibility under the complex carbon source condition.

**Example 4. Identification of novel *Schizochytrium* sp. strain CD01-1821**

For biomolecular identification of the separated and selected microalgal strain CD01-1821 in Example 1 and Example 2, the 18S rRNA gene sequence was analyzed.

Specifically, after extracting and separating gDNA from the colony of the pure separated microalgae CD01-1821, PCR amplification reaction was performed using primers 18s-Fwd, LABY-ARev for gene amplification of the 18s rRNA region described in Table 4.

[Table 4]

| SEQ ID NO: | Primer | Sequence (5' - 3') |
|---|---|---|
| 1 | 18S-Fwd | AAC CTG GTT GAT CCT GCC AGT |
| 2 | LABY-ARev | GGG ATC GAA GAT Are TAG |

For PCR reaction, using a reaction solution containing taq polymerase, denaturation at 95°C for 5 minutes was performed, and then denaturation at 95°C for 30 seconds, annealing at 50°C for 30 seconds, and polymerization at 72°C for 2 minutes were repeated 35 times, and then polymerization reaction was performed at 72°C for 5 minutes. The reaction solution amplified through the PCR process was under electrophoresis in 1% agarose gel, and thereby, it was confirmed that DNA fragments of about 1000 bp size were amplified, and nucleotide sequence sequencing analysis was progressed. As the result of analysis, it was confirmed that the obtained corresponding sequence showed homology of 95.11% to the 18S rRNA gene sequence of *Schizochytrium limacinum* strain OUC109 belonging to Thraustochytrid family (科) series microalgae, and showed homology of 95.0% to the 18S rRNA gene sequence of *Schizochytrium* sp. strain LY-2012 of *Schizochytrium* sp., through NCBI BLAST search. Through this, it was confirmed that the isolated microalgae CD01-1821 was a novel *Schizochytrium* sp. strain, and named *Schizochytrium* sp. CD01-1821 strain, and deposited at Korea Research Institute of Bioscience and Biotechnology, Korean Collection for Type Cultures (KCTC) on August 23, 2021 and was given an accession number KCTC14660BP.

**Example 5. Analysis of crude protein content of CD01-1821 strain culturing solution samples**

In order to analyze the crude protein content in the *Schizochytrium* sp. CD01-1821 strain culturing solution samples, the method as below was used.

Specifically, dried microbial cells, each fermented solution dried product (specimen) corresponding to about 20-30 mg was precisely measured and placed in a decomposition tube, and two decomposition accelerators were added. The decomposition accelerator is effectively decomposed when the ratio of sulfuric acid ($H_2SO_4$) and potassium sulfate ($K_2SO_4$) is 1.4-2.0:1.0. Then, concentrated sulfuric acid ($H_2SO_4$) 12-15 mL was added to the decomposition tube, and it was cooled to a room temperature when the color of the decomposed solution was transparent light green (using a copper accelerator) or transparent yellow (using a selenium catalyst) by decomposing in a decomposition device at 420°C for 45 to 60 minutes. After cooling, 80mL of distilled water was added to the decomposed test solution. After adding 25 mL of collection solution mixed with the mixing indicator into an Erlenmeyer flask, this was placed on a distillation apparatus, and the Erlenmeyer flask stand was lifted so that the distilled solution entered the collection solution during distillation. 50 mL of sodium hydroxide solution (NaOH) (amount corresponding to 4 times of sulfuric acid used during decomposition) was added to a decomposition tube, and it was distilled in a distillation apparatus for 3 to 4 minutes. It was confirmed that the collection solution in the Erlenmeyer flask of the distillation apparatus turned green while collecting ammonia ($NH_3$) contained in the distilled solution. The distilled solution was titrated using a hydrochloric acid solution (generally, 0.1N or 0.2N) until the end point reached a pale pink color, and the amount of acid used for titration was recorded. In case of an automatic device, distillation, titration and calculation are all performed automatically. Using the experimental result, nitrogen % was derived by Calculation formula 2 below. Protein quantification was expressed by multiplying the previously derived nitrogen % by the average nitrogen coefficient of 6.25.

Nitrogen (%) = {(HCl amount mL - blank test mL) $\times$ M $\times$ 14.01/specimen amount mg} $\times$ 100     [Calculation formula 2]

* 14.01: Atomic weight of nitrogen
* M: Molarity of HCl
* Degradation accelerator: Kjeltabs or equivalent one thereto
* Boric acid solution: 1% (or 4%) boric acid solution made to a constant volume of 10 L by adding $H_3BO_3$ 100 g (or 400 g), 0.1% bromocresol green solution 100 mL and 0.1% methyl red solution 100 mL

For amino acid content analysis, a fermented solution dried product (specimen) sample of about 1g was collected from the culturing solution of the CD01-1821 and CD01-2147 strains. After progressing acid hydrolysis of intracellular protein utilizing HCl solution at a concentration of 6N, it was diluted and filtered with distilled water and liquid chromatography was performed.

[Table 5]

|  | *Schizochytrium* sp. CD01-1821 |
|---|---|
| Crude protein amount (%) | 17 |
| Amino acid amount (mg/L) |  |
| Aspartic acid | 78.44 |
| Threonine | 0 |
| Serine | 108.65 |
| Glutamic acid | 790.8 |
| Glycine | 165.08 |
| Alanine | 172.72 |
| Cysteine | 0 |
| Valine | 96.35 |
| Methionine | 46.54 |
| Isoleucine | 16.88 |
| Leucine | 13.17 |
| Tyrosine | 229.64 |
| Phenylalanine | 166.55 |
| Lysine | 85.83 |

(continued)

|  | *Schizochytrium* sp. CD01-1821 |
|---|---|
| Histidine | 0 |
| Arginine | 114.15 |

As a result, as shown in Table 5, it was confirmed that the crude protein content in the CD01-1821 dried microbial cell was 17%. In addition, in the amino acid content in the dried microbial cell, glutamic acid was highest, and it was high in the order of tyrosine, alanine, phenylalanine, glycine, arginine, serine, valine, lysine, aspartic acid, methionine, isoleucine and leucine. Through this, it was confirmed that the amino acid in the CD01-1821 dried microbial cell was composed of glutamic acid, tyrosine, alanine, phenylalanine, glycine, arginine, serine, valine, lysine, aspartic acid, methionine, isoleucine and leucine.

From the above description, those skilled in the art to which the present application pertains will be able to understand that the present application may be implemented in other specific forms without changing the technical spirit or essential characteristics thereof. In this regard, it should be understood that the examples described above are illustrative and not restrictive in all respects. The scope of the present application should be construed as including all changes or modified forms derived from the meaning and scope of the claims to be described later and equivalent concepts thereof rather than the detailed description.

[Accession Number]

Name of Depository Authority: Korea Research Institute of Bioscience and Biotechnology Korea Collection for Type Culture (KCTC)
Accession number: KCTC14660BP
Date of Deposit: 20210823

# EP 4 431 594 A1

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

# RECEIPT IN THE CASE OF AN ORIGINAL DEPOSIT

issued pursuant to Rule 7.1

TO: **CJ CheilJedang Corporation**

CJ CheilJedang Corporation

330, Dongho-ro, Jung-gu, Seoul

Republic of Korea

| I. IDENTIFICATION OF THE MICROORGANISM | |
|---|---|
| Identification reference given by the DEPOSITOR: <br><br>***Schizochytrium* sp. CD01-1821** | Accession number given by the INTERNATIONAL DEPOSITARY AUTHORITY: <br><br>**KCTC 14660BP** |

| II. SCIENTIFIC DESCRIPTION AND/OR PROPOSED TAXONOMIC DESIGNATION |
|---|
| The microorganism identified under I above was accompanied by: <br><br> [ ] a scientific description <br><br> [ ] a proposed taxonomic designation <br><br> (Mark with a cross where applicable) |

| III. RECEIPT AND ACCEPTANCE |
|---|
| This International Depositary Authority accepts the microorganism identified under I above, which was received by it on **August 23, 2021.** |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: **Korean Collection for Type Cultures** <br><br> Address: Korea Research Institute of Bioscience and Biotechnology (KRIBB) 181, Ipsin-gil, Jeongeup-si, Jeolllabuk-do 56212 Republic of Korea | Signature(s) of person(s) having the power to represent the International Depositary Authority or of authorized official(s): <br><br> *Song-Gun Kim* <br><br> KIM, Song-Gun, Director <br> Date: **August 23, 2021** |

Form BP/4 (KCTC Form 17)                                             sole page

**Claims**

1. A novel *Schizochytrium* sp. CD01-1821 microalgae (Accession number KCTC14660BP).

2. The *Schizochytrium* sp. microalgae according to claim 1, wherein the *Schizochytrium* sp. CD01-1821 microalgae have a producing ability of omega-3 unsaturated fatty acid.

3. The *Schizochytrium* sp. microalgae according to claim 2, wherein the omega-3 unsaturated fatty acid is docosahexaenoic acid (DHA) and eicosapentaenoic acid (EPA).

4. The *Schizochytrium* sp. microalgae according to claim 3, wherein the microalgae produce docosahexaenoic acid of 35 to 60 % by weight based on the total weight of the fatty acid.

5. The *Schizochytrium* sp. microalgae according to claim 3, wherein the microalgae produce eicosapentaenoic acid of 0.1 to 2 % by weight based on the total weight of the fatty acid.

6. Biomass derived from a *Schizochytrium* sp. microalgae, comprising the *Schizochytrium* sp. microalgae of claim 1, a culture of the microalgae, a dried product of the culture, or a lysate of the dried product.

7. A composition comprising the biomass derived from a *Schizochytrium* sp. microalgae of claim 6, a concentrate or a dried product of the biomass, or an extract of the biomass.

8. The composition according to claim 7, wherein the composition is a feed composition or a food composition.

9. A method for producing biomass derived from a *Schizochytrium* sp. microalgae, comprising:

    1) culturing the *Schizochytrium* sp. CD01-1821 microalgae of claim 1; and
    2) recovering biomass containing docosahexaenoic acid from the microalgae, a dried product thereof, or a lysate thereof.

10. The method for producing biomass derived from a *Schizochytrium* sp. microalgae according to claim 9, wherein the culturing is performed under a heterotrophic condition.

11. The method for producing biomass according to claim 9, wherein the culturing is performed using a medium comprising a carbon source and a nitrogen source.

12. The method for producing biomass according to claim 11, wherein the carbon source is one or more kinds selected from the group consisting of glucose, fructose, maltose, galactose, mannose, sucrose, arabinose, xylose and glycerol.

13. The method for producing biomass according to claim 11, wherein the nitrogen source is i) any one or more of organic nitrogen sources selected from the group consisting of yeast extract, beef extract, peptone and tryptone, or ii) any one or more of inorganic nitrogen sources selected from the group consisting of ammonium acetate, ammonium nitrate, ammonium chloride, ammonium sulfate, sodium nitrate, urea and MSG (Monosodium glutamate).

14. A method for producing bio-oil derived from a *Schizochytrium* sp. microalgae, comprising:

    1) culturing the *Schizochytrium* sp. CD01-1821 microalgae of claim 1; and
    2) recovering biomass containing docosahexaenoic acid from the microalgae, a dried product thereof, or a lysate thereof.

【FIG. 1】

【FIG. 2】

【FIG. 3】

## INTERNATIONAL SEARCH REPORT

| International application No. |
| --- |
| **PCT/KR2022/011959** |

**A. CLASSIFICATION OF SUBJECT MATTER**

**C12N 1/12**(2006.01)i; **C12P 7/40**(2006.01)i; **A23K 10/16**(2016.01)i; **C11B 1/02**(2006.01)i; C12R 1/89(2006.01)n

According to International Patent Classification (IPC) or to both national classification and IPC

**B. FIELDS SEARCHED**

Minimum documentation searched (classification system followed by classification symbols)

C12N 1/12(2006.01); A23K 10/16(2016.01); C12N 1/00(2006.01); C12P 23/00(2006.01); C12P 7/20(2006.01); C12P 7/64(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 스키조키트리움(Schizochytrium), KCTC14660BP, 오메가-3 불포화 지방산 (omega-3 unsaturated fatty acid), 도코사헥사엔산(docosahexaenoic acid, DHA), 에이코사펜타엔산(eicosapentaenoic acid, EPA)

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-1847551 B1 (INDUSTRIAL COOPERATION FOUNDATION JEONBUK NATIONAL UNIVERSITY et al.) 10 April 2018 (2018-04-10)<br>See claims 1 and 6-8; paragraphs [0053] and [0057]-[0060]; and table 2. | 1-14 |
| X | PARK, H. et al. Enhanced production of carotenoids using a Thraustochytrid microalgal strain containing high levels of docosahexaenoic acid-rich oil. Bioprocess and Biosystems Engineering. 2018, vol. 41, pp. 1355–1370.<br>See abstract; pages 1356-1357; and tables 1-2 and 5. | 1-14 |
| X | KR 10-2008-0111586 A (LEE, Joung Yeoul) 24 December 2008 (2008-12-24)<br>See claims 1-3; and table 1. | 1-14 |
| A | KR 10-2017-0032577 A (KOREA RESEARCH INSTITUTE OF BIOSCIENCE AND BIOTECHNOLOGY) 23 March 2017 (2017-03-23)<br>See entire document. | 1-14 |

| ✓ Further documents are listed in the continuation of Box C. | ✓ See patent family annex. |
| --- | --- |

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **14 November 2022** | **14 November 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2022/011959** |

**C. DOCUMENTS CONSIDERED TO BE RELEVANT**

| Category* | Citation of document, with indication, where appropriate, of the relevant passages | Relevant to claim No. |
| --- | --- | --- |
| A | KR 10-2019-0110186 A (ADVANCED BIOMASS R&D CENTER) 30 September 2019 (2019-09-30)<br>See entire document. | 1-14 |
| PX | KR 10-2022-0080594 A (CJ CHEILJEDANG CORPORATION) 14 June 2022 (2022-06-14)<br>See claims 1-3 and 7-14. | 1-14 |

Form PCT/ISA/210 (second sheet) (July 2022)

<table>
<tr><td rowspan="2"><b>INTERNATIONAL SEARCH REPORT</b></td><td>International application No.</td></tr>
<tr><td><b>PCT/KR2022/011959</b></td></tr>
</table>

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

| | | International application No. |
|---|---|---|
| | | **PCT/KR2022/011959** |

| Patent document<br>cited in search report | Publication date<br>(day/month/year) | Patent family member(s) | | Publication date<br>(day/month/year) |
|---|---|---|---|---|
| KR 10-1847551 B1 | 10 April 2018 | None | | |
| KR 10-2008-0111586 A | 24 December 2008 | None | | |
| KR 10-2017-0032577 A | 23 March 2017 | KR 10-1777217 | B1 | 11 September 2017 |
| KR 10-2019-0110186 A | 30 September 2019 | None | | |
| KR 10-2022-0080594 A | 14 June 2022 | WO 2022-124590 | A1 | 16 June 2022 |

Form PCT/ISA/210 (patent family annex) (July 2022)

**REFERENCES CITED IN THE DESCRIPTION**

**Patent documents cited in the description**

- KR 1020210152561
- US 5130242 A

- KR 102100650 B1

**Non-patent literature cited in the description**

- **MIAO, X ; WU, Q.** *Biosource Technology,* 2006, vol. 97, 841-846